# EUROPEAN PATENT SPECIFICATION

(11) **EP 0 466 705 B1**
(45) Date of publication and mention of the grant of the patent: **12.06.1996**
(21) Application number: 90903661.8
(22) Date of filing: 12.02.1990
(51) Int. Cl.: A61M 3/02

(54) **METHOD AND APPARATUS FOR OCULAR PERFUSION**
VERFAHREN UND VORRICHTUNG ZUR OKULAREN PERFUSION
PROCEDE ET APPAREIL DE PERFUSION OCULAIRE

(43) Date of publication of application: 22.01.1992
(73) Proprietor: Alcon Laboratories, Inc., Fort Worth Texas 76124-2099 (US)
(72) Inventor: MORRIS, Robert, E., Birmingham, AL 35213 (US); WITHERSPOON, Clark, Douglas, Birmingham, AL 35213 (US); GOGGANS, William, E., Jr., Pinson, AL 35126 (US)
(74) Representative: Senior, Alan Murray
(86) International application number: PCT/US90/00800
(87) International publication number: WO 91/12034

(56) References cited:
- EP-A- 0 121 277
- EP-A- 0 254 658
- EP-A- 0 348 146
- US-A- 4 184 510
- US-A- 4 331 130
- US-A- 4 813 927
- PATENT ABSTRACTS OF JAPAN, vol. 13, no. 516 (C-656) 17 November 1989 & JP-A-12 009 054 (HITACHI LTD) 22 August 1989

## Description

The present invention relates generally to apparatus used in surgery conducted on the eye. More particularly, the present invention relates to apparatus used in continuous infusion processes associated with such surgery. In even greater particularity, the present invention relates to apparatus for accurately and rapidly controlling the infusion pressure to the eye and rapidly converting between liquid and gas infusion during such surgery.

Intraoperative control of intraocular infusion pressure is an important parameter in eye surgery. Liquid pressure regulation has been accomplished in most part using gravity-fed systems involving the relative height of the infusion bottle above the eye. A discussion of the development of gas infusion may be found in "Vitreous Microsurgery" by Steven Charles, M.D. in Williams & Wilkins, 1981, volume 4. As noted therein, there are known power injectors or pumps which are capable of maintaining an accurate intraocular pressure during air infusion, as compared to manual syringe injection. Such devices have also been developed wherein a microcompressor is used to produce an inflow of gas dependent upon intraocular pressure.

As these advances are made in gas infusion apparatus, a need exists for controlled intraocular infusion method and apparatus which would facilitate the interchangeability of gas infusion and liquid infusion during surgery on the posterior segment of the eye (vitrectomy). Additionally, during surgery on the anterior chamber of the eye (cataract extraction) the need exists for more accurate, surgeon controlled and monitored liquid infusion.

During cataract surgery, the most common operation performed in the United States, the surgeon views only the anterior chamber of the eye as shown in FIG. 5, having no method of simultaneously monitoring the posterior segment.

Since the anterior chamber is quite small, containing only 0.25cc of liquid volume, small and momentary aspiration flow rates exceeding infusion rates will result in anterior chamber collapse, as shown in FIG. 5. The consequence of anterior chamber collapse is damage to the nonreproducible monolayer of cells (endothelium) which lines the inner surface of the cornea, keeping it clear of fluid. This endothelial damage can then result in clouding of the cornea, with the need for subsequent cornea transplantation.

It is common for cataract surgeons to perform incomplete temporary closure of the surgical incision prior to irrigation/aspiration cataract cortex removal. A true "closed-eye" system is never achieved, and anterior chamber collapse in this condition is a sign of wound leakage of infusion liquids rather than of inadequate infusion pressure. If anterior chamber collapse is encountered, the wound should be checked for tightness.

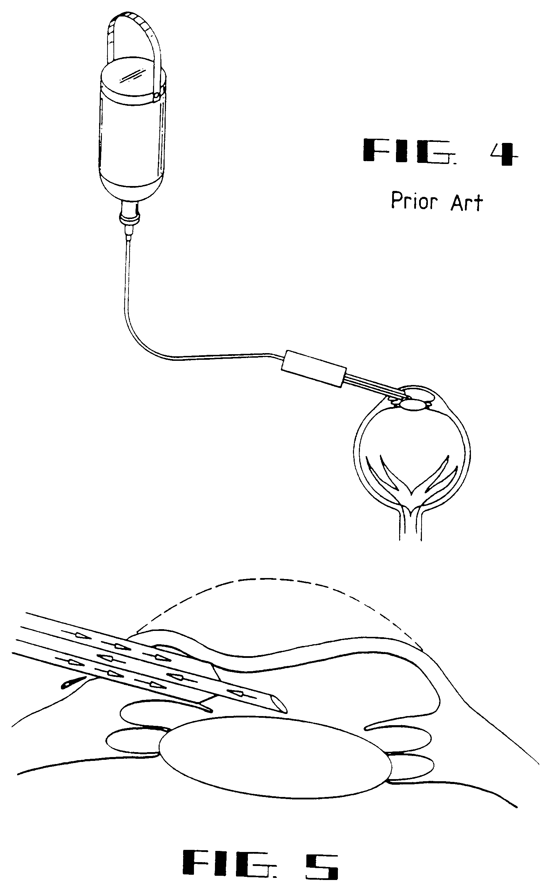

The cataract surgeon's most common defense against perceived anterior collapse is increasing of infusion pressure by raising a gravity-feed infusion bottle an estimated height above the eye as shown i n FIG. 4. Note that gas is not commonly infused into the anterior chamber in such surgery. In practice, bottle height is not measured, so that actual pressure delivered to the eye is unknown, and is presumed to be sufficient when anterior chamber collapse no longer occurs.

Unfortunately, corneal endothelial damage may also occur as a result of high infusate volume or jetstream mechanical damage from the use of high flow rates under high pressure. These effects, moreover, are not readily apparent to the surgeon due to his inability to perceive the flow rate or pressure. Retinal artery occlusion may also occur, invisible to the surgeon and resulting in blindness. Finally, iris prolapse through the wound may occur as a result of excessive infusion pressure.

Ideally, cataract irrigation/aspiration surgery should be performed with normal ocular pressure (25mm Hg). Failing this, infusion pressure should be raised as little as possible to maintain anterior chamber pressure to avoid collapse during active irrigation/aspiration. Because unnecessarily high infusion pressure and flow rates can injure retinal and corneal tissues with little warning to the surgeon, increasing infusion to the eye should be the last solution attempted to remedy anterior chamber collapse. As previously stated, the surgeon should first check adequate wound closure. Further, even with an adequately closed eye, anterior chamber collapse may occur as a result of undisciplined, continuous, high rate aspirations. High vacuum aspiration is necessary to achieve cataract removal, but successful removal of blocks of cataract cortex material (with subsequent opening of the aspiration port) should be anticipated so as to intermittently reduce the aspiration vacuum, avoiding anterior chamber collapse. All too commonly surgeons move about within the eye with an open aspiration port, with suction continuously engaged.

The above discussion demonstrates the importance of a new method of rapid, accurate, surgeon-controlled ocular infusion (Gas Forced Liquid Infusion, GFLI).

US-A-4 813 927 discloses apparatus for intraocular infusion in accordance with the precharacterising portion of claim 1.

EP-A-0 121 277 discloses similar apparatus in which gas is used to pressurise liquid delivery to an intraocular surgical device.

EP-A2-0 254 658 discloses a support for use in surgery to hold or move a limb. It may be provided with electrical motors operable by voice commands.

The primary object of the invention is to provide the surgeon with apparatus to rapidly and precisely control and monitor the infusion pressure.

According to the present invention, there is provided a Gas Forced Liquid Infusion (GFLI) apparatus for controlling intraocular pressure during closed wound intraocular surgery, using a source of continuous pressurised gas controllable to vary gas pressure, a liquid infusate reservoir having a limited volume for maintaining a liquid infusate in the reservoir under pressure with a volume of gas, and an ocular surgical infusion instrument, the apparatus comprising:
a first conduit for conveying pressurised gas from the source of continuous pressurised gas to the liquid infusate reservoir,
a second conduit for conveying only liquid infusate from the reservoir to the ocular surgical infusion instrument;
a third conduit for conveying pressurised gas from the source of continuous pressurised gas to the ocular surgical infusion instrument;
a valving means in communication with the second and third conduits for selectively connecting either the second or third conduit to the ocular surgical infusion instrument such that when the second conduit is connected to the ocular surgical infusion instrument, liquid infusate from the liquid infusate reservoir is conveyed to the ocular surgical infusion instrument, and when the third conduit is connected to the surgical infusion instrument, pressurised gas from the source of continuous pressurised gas is conveyed to the infusion instrument;
characterised in that the conduit for conveying the pressurised gas extends into the volume of gas contained within the reservoir so that the volume of gas contained within the reservoir is in direct communication with the source of continuous pressurised gas, thereby permitting accurate and rapid control of infusion pressure during intraocular surgery and avoiding the deleterious consequences of underpressurisation or overpressurisation and jetstreaming during such surgery.

In one embodiment, the invention, through a novel arrangement of conduits and valves, allows the constant maintenance of the desired intraocular pressure and the flexibility of using either gas or liquid infusion. The invention also enables the usage of monetary maximum safe infusion pressure for such purposes as controlling bleeding during surgery.

The invention may utilise an infusion bottle as a reservoir for the infusion liquid. A continuous infusion gas pump, such as a Grieshaber or Trek Air Pump, may be utilised as the source to pressurise the liquid infusion bottle to the desired infusion pressure (Gas Forced Liquid Infusion, GFLI). The output of the pump may also be used directly to provide pressurised gas via a conduit for gas infusion to the eye. In that case, the gas conduit and fluid conduit are preferentially formed as a dual-tube conduit and are connected to a stopcock which allows the physician to quickly select between either gas infusion or liquid infusion.

Apparatus embodying features of our invention are depicted in the appended drawings which form a portion of this invention and wherein:
FIG. 1 is a perspective view showing the apparatus as used during surgery;
FIG. 2 is a broken lay-out view showing the invention not in use;
FIG. 3 is a broken lay-out view showing the cataract surgery embodiment;
FIG. 4 shows the prior art and ocular structures; and
FIG. 5 shows an instance of ocular collapse.

FIG. 4 illustrates the common control methodology and apparatus for liquid infusion of the eye as heretofore used in cataract and in vitreous surgery. FIG. 5 is illustrative of common ocular collapse when using the method and apparatus shown in FIG. 4.

Referring to the figures for a better understanding of the invention, it may be seen that the invention is for use with a patient who is lying beneath surgical drapes 11 on an operating table 12. A continuous infusion air/gas pump 13 is located on an equipment stand 14 as is conventional practice. The output of the air/gas pump 13 is displayed as by LED's at a panel 16 so that the pressure generated thereby may be monitored by the surgery team and precisely controlled.

The output of the air/gas pump 13 is used to pressurize a liquid infusion bottle 23 to provide Gas Forced Liquid Infusion (GFLI) of the eye. Use of Gas Forced Liquid Infusion (with digital numeric readout of infusion pressure in view of the surgeon) allows the surgeon for the first time to accurately and continuously monitor this most important parameter. In this method, the infusion bottle is hung at eye height, so that gravity feed, the preferred technique of the prior art, contributes no pressure to perfusion/infusion. Rather, total control of perfusion pressure is instead achieved by instilling in the infusate bottle, gas under pressure, provided by the gas pump. The infusion pressure can thereby be meticulously and rapidly selected by the surgeon.

An antibacterial filter connector 17 attaches a flexible conduit to the pump 13 in any conventional manner as is well known in the art. The conduit 18 extends from the equipment stand 14 to near an IV support 19 and terminates in a T-connector 21 or other suitable device for directing the airflow from the pump 13 along two paths. A fluid pressurization conduit 22 is connected between the T-connector 21 and a combined infusion bottle with drip chamber 23 supported by the IV support 19 and serves to pressurize the fluid therein in accordance with the output pressure of the pump 13. This fluid pressurization conduit extends within the infusion bottle so as to connect the gas volume above liquid with the gas pump for venting purposes. A fluid delivery conduit 24 is connected as the output of infusion bottle 23 and terminates as one input to infusate stopcock selection valve 26. An air delivery conduit 27 is connected between the remaining branch of the T-connector 21 and the valve 26. Note that valve 26 may be a three-way input valve which would allow selection of either liquid, air, or finally an air/gas mixture (e.g. sulfur hexafluoride SF₆ 20%, perfluoropropane C₃F₈ 15%) delivered by a second gas pump. The valve 26 has a single output to an infusion conduit 28 which is connected to and supplies an eye infusion cannula 29.

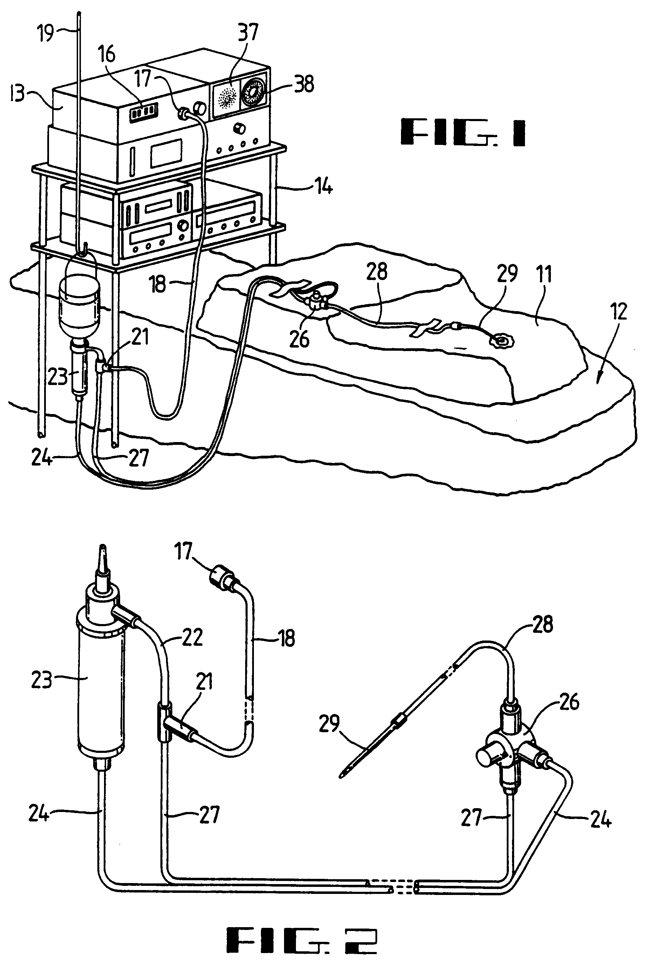

As shown in FIG. 1, the infusion bottle 23 is placed at the patient's eye level so as to contribute no gravity infusion pressure as had been customary in the prior art. Air/gas is pumped into the bottle 23 via conduits 18 and 22 to provide the desired infusion pressure. Although a normal starting pressure may be selected, it should be clear that the infusion pressure can be rapidly changed by adjusting the output pressure of the air pump 13. The air pump selected should have a digital display 16 of the pressure which should be visible to all operating room personnel. It has been determined that the displayed, conduit, and intraocular static pressures agree to within two to four mm of Hg using the present apparatus.

Valve 26 allows the surgical team to quickly switch from liquid infusion to gas infusion. Conduits 24 and 27 may be formed from the two halves of a twin plastic tube, for example Dicoc Twin Bore Silicone IV tubing, such that the valve 26 may be located immediately proximal to the short cannula 29, thereby minimizing the time and volume required to clear infusion liquid from the system cannula 29 when gas is desired.

From the foregoing, it may be seen that we have provided an effective apparatus and method (Gas Forced Liquid Infusion, GFLI) which greatly improves the surgeon's efficiency in vitrectomy operations in which liquid to gas infusion changes are desired and also provides a readily controllable means for varying the infusion pressure during all liquid infusion eye surgery including cataract removal. As is well known, it is possible to stop or reduce bleeding by raising the intraocular pressure to maximum known safe levels, usually 35 to 45 mm of Hg. Using the present invention with a digital display 16 allows the surgical team to quickly determine the infusion pressure levels and rapidly change the level as required, to maximum safe level with great accuracy.

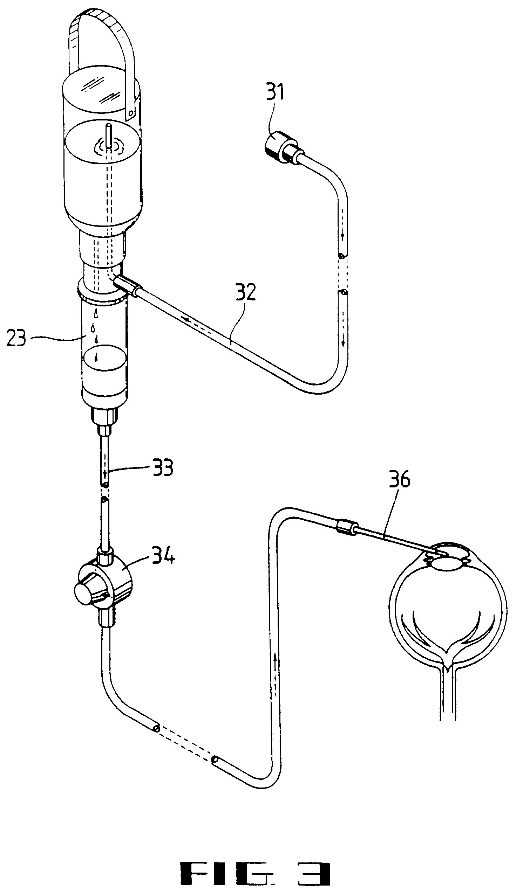

It is the aspect of the invention that allows the surgical team to continuously monitor and precisely control the infusion pressure which is of critical importance to the cataract surgeon. The tubing system shown in FIG. 3 uses an antibacterial filter connector 31 to attach a flexible tubing 32 to the air pump 13. The flexible tubing 32 is connected directly to the infusion bottle 23 which is supplied with infusion liquid as shown in FIG. 2. A single flexible conduit 33 is provided to carry pressurized infusion liquid from the bottle to a valve 34 which controls the flow of liquid to the infusion/aspiration device 36 used in cataract surgery. With the digital readout 16 available, the surgeon is able to constantly monitor the pressure being utilized in the eye and therefore is at all times aware of and alert to the potential deleterious consequences of overpressurization and jet-streaming. Thus, due to his ability to monitor and accurately select the intraocular pressure, the surgeon will naturally turn more attention to adequate wound closure and disciplined aspiration rather than using a potentially destructive infusion pressure level to prevent ocular collapse.

The use of the gas pump 13 to pressurize the infusion bottle 23 in either of the embodiments above also leads to a significant further refinement in the art. Voice recognition technology can be used to regulate the output pressure of the gas pump 3. Thus, an input microphone 37 is connected to a voice recognition circuit, many of which are commercially available, which in turn outputs a control signal to the pump 13. Preferentially the pump will be provided with a speaker 38 which will enunciate the pressure, subsequent to an instruction to change pressure or upon a query by the surgeon; or upon a variance of the pressure outside a predetermined tolerance. The surgeon's voice may be specifically recognized so that he might state the desired pressure in an audible voice, and the machine would respond immediately that it will seek the commanded pressure after a preset safety delay, in the absence of further commands. Infusate selector valve 26 may also be voice actuated by having valve position audible control means connected thereto. Feedback means for acknowledging response of the valve position audible control means may also be provided. For the first time, perfusion pressure to the eye and infusate source can be controlled directly by the surgeon rather than necessitating the presence of other operating room personnel. Immediate pressure adjustment with voice response completes surgeon control of infusion pressure - the most vital parameter characterising "closed-eye" surgery.

## Claims

1. A Gas Forced Liquid Infusion (GFLI) apparatus for controlling intraocular pressure during closed wound intraocular surgery, using a source (13) of continuous pressurised gas controllable to vary gas pressure, a liquid infusate reservoir (23) having a limited volume for maintaining a liquid infusate in the reservoir (23) under pressure with a volume of gas, and an ocular surgical infusion instrument (29;36), the apparatus comprising:
a first conduit (18;32) for conveying pressurised gas from the source (13) of continuous pressurised gas to the liquid infusate reservoir (23),
a second conduit (23;33) for conveying only liquid infusate from the reservoir (23) to the ocular surgical infusion instrument (29;36);
a third conduit (27) for conveying pressurised gas from the source (13) of continuous pressurised gas to the ocular surgical infusion instrument (29);
a valving means (26;34) in communication with the second and third conduits (23;33;27) for selectively connecting either the second (24) or third (27) conduit to the ocular surgical infusion instrument (29) such that when the second conduit (24) is connected to the ocular surgical infusion instrument (29), liquid infusate from the liquid infusate reservoir (23) is conveyed to the ocular surgical infusion instrument (29), and when the third conduit (27) is connected to the surgical infusion instrument (29), pressurised gas from the source (13) of continuous pressurised gas is conveyed to the infusion instrument (29);
characterised in that the conduit (18;32) for conveying the pressurised gas extends into the volume of gas contained within the reservoir (23) so that the volume of gas contained within the reservoir (23) is in direct communication with the source (13) of continuous pressurised gas, thereby permitting accurate and rapid control of infusion pressure during intraocular surgery and avoiding the deleterious consequences of underpressurisation or overpressurisation and jetstreaming during such surgery.

2. Apparatus according to claim 1, further comprising:
a gas pressure control means (27) for variably controlling the pressure of the gas from the source (13) of continuous pressurised gas by audible command signals.

3. An apparatus according to claim 2, further comprising an indication means (38), in connection with said gas pressure control means (27), for providing a human audible indication of the pressure of the gas from the source of continuous pressurised gas (13).

4. An apparatus according to claim 2, wherein said gas pressure control means (37) for variably controlling the pressure of the gas from the source (13) of continuous pressurised gas provides an audible signal acknowledging a detected audible command signal.

5. Apparatus according to claim 4, further comprising a valve position audible-control means connected to the valving means (26) for selectively controlling the connection of either the second (24) or third (27) conduit to the ocular surgical infusion instrument (29) by audible command signals.

6. Apparatus according to claim 5, further comprising a feedback means for acknowledging response of the valve position audible-control means.

## Patentansprüche

1. Eine Vorrichtung zur gasgesteuerten Flüssigkeitsinfusion (GFLI(= Gas Forced Liquid Infusion)-Vorrichtung) zum Steuern des Augeninnendrucks während einer intraokularen Operation bei geschlossener Wunde, bei der eine Permanent-Druckgasquelle (13), die dahingehend gesteuert werden kann, den Gasdruck zu variieren, ein Flüssiginfusatreservoir (23) mit einem begrenzten Volumen, um ein flüssiges Infusat in dem Reservoir (23) mit einem Gasvolumen unter Druck stehend zu halten, und ein Augenchirurgie-Infusionsinstrument (29;36) verwendet werden, wobei die Vorrichtung folgendes umfaßt:
eine erste Leitung (18;32) zum Befördern von Druckgas von der Permanent-Druckgasquelle (13) zu dem Flüssiginfusatreservoir (23),
eine zweite Leitung (23;33) zum Befördern nur des Flüssiginfusats von dem Reservoir (23) zu dem Augenchirurgie-Infusionsinstrument (29;36),
eine dritte Leitung (27) zum Befördern von Druckgas von der Permanent-Druckgasquelle (13) zu dem Augenchirurgie-Infusionsinstrument (29);
ein Ventilmittel (26;34), das mit den zweiten und dritten Leitungen (23;33;27) in Verbindung steht, um wahlweise entweder die zweite (24) oder die dritte (27) Leitung mit dem Augenchirurgie-Infusionsinstrument (29) derart zu verbinden, daß dann, wenn die zweite Leitung (24) mit dem Augenchirurgie-Infusionsinstrument (29) verbunden ist, das flüssige Infusat von dem Flüssiginfusatreservoir (23) zu dem Augenchirurgie-Infusionsinstrument (29) befördert wird, und daß dann, wenn die dritte Leitung (27) mit dem chirurgischen Infusionsinstrument (29) verbunden ist, das Druckgas von der Permanent-Druckgasquelle (13) zu dem Infusionsinstrument (29) befördert wird,
dadurch gekennzeichnet, daß sich die Leitung (18;32) zum Befördern des Druckgases in den Gasraum erstreckt, der in dem Reservoir (23) enthalten ist, so daß der Gasraum, der in dem Behälter (23) vorhanden ist, in direkter Verbindung mit der Permanent-Druckgasquelle (13) steht, wodurch eine genaue und schnelle Steuerung des Infusionsdrucks während einer intraokularen Operation möglich ist und die nachteiligen Folgen einer Unterdruckbeaufschlagung oder einer Überdruckbeaufschlagung und das Strömen eines Strahls während einer derartigen Operation vermieden werden können.

2. Vorrichtung nach Anspruch 1, desweiteren mit:
einem Gasdrucksteuermittel (27) zum variablen Steuern des Drucks des Gases von der Permanet-Druckgasquelle (13) durch akustische Befehlssignale.

3. Vorrichtung nach Anspruch 2, desweiteren mit einem Anzeigemittel (38) in Verbindung mit dem Gasdrucksteuermittel (27) zum Liefern einer für Menschen hörbaren Anzeige des Drucks des Gases von der Permanet-Druckgasquelle (13).

4. Vorrichtung nach Anspruch 2, bei der das Gasdrucksteuermittel (27) zum variablen Steuern des Drucks des Gases von der Permanet-Druckgasquelle (13) ein akustisches Signal liefert, das ein erfaßtes akustisches Befehlssignal bestätigt.

5. Vorrichtung nach Anspruch 4, desweiteren mit einem akustischen Ventilstellungssteuerungsmittel, das mit dem Ventilmittel (26) verbunden ist, um wahlweise die Verbindung entweder der zweiten (24) oder der dritten (27) Leitung mit dem Augenchirurgie-Infusionsinstrument (29) durch akustische Befehlssignale zu steuern.

6. Vorrichtung nach Anspruch 5, desweiteren mit einem Feedback-Mittel zum Bestätigen der Reaktion des akustischen Ventilstellungssteuerungsmittels.

## Revendications

1. Appareil d'infusion de liquide sous pression de gaz (GFLI) pour commander la pression intraoculaire durant une intervention chirurgicale intraoculaire à incision fermée, utilisant une source (13) de gaz pressurisé en permanence pouvant être commandée pour modifier la pression du gaz, un réservoir de liquide d'infusion (23) ayant un volume limité pour maintenir un liquide d'infusion dans le réservoir (23) sous pression avec un volume de gaz, et un instrument d'infusion chirurgicale oculaire (29; 36), appareil comportant :
un premier conduit (18; 32) pour acheminer le gaz pressurisé depuis la source (13) de gaz sous pression permanente au réservoir de liquide d'infusion (23),
un second conduit (24; 33) pour acheminer seulement le liquide d'infusion depuis le réservoir (23) vers l'instrument d'infusion chirurgicale oculaire (29; 36);
un troisième conduit (27) pour acheminer le gaz sous pression depuis la source (13) de gaz pressurisé en permanence vers l'instrument d'infusion chirurgicale oculaire (29);
un moyen de distribution (26; 34) en communication avec les second et troisième conduits (24; 33; 27) pour relier sélectivement soit le second (24), soit le troisième (27) conduit à l'instrument d'infusion chirurgicale oculaire (29) de telle sorte que, lorsque le second conduit (24) est relié à l'instrument d'infusion chirurgicale oculaire (29), le liquide d'infusion provenant du réservoir de liquide d'infusion (23) est acheminé vers l'instrument d'infusion chirurgicale oculaire (29), et lorsque le troisième conduit (27) est relié à l'instrument d'infusion chirurgicale (29), du gaz pressurisé provenant de la source (13) de gaz pressurisé permanente est acheminé vers l'instrument d'infusion (29);
caractérisé en ce que le conduit (18; 32) pour acheminer le gaz pressurisé s'étend dans le volume de gaz contenu à l'intérieur du réservoir (23) de sorte que le volume de gaz contenu à l'intérieur du réservoir (23) se trouve en communication directe avec la source (13) de gaz pressurisé permanente, permettant ainsi une commande précise et rapide de pression d'infusion durant une chirurgie intraoculaire et évitant les conséquences néfastes de dépressurisation ou de surpressurisation et un écoulement sous forme de jet durant cette chirurgie.

2. Appareil selon la revendication 1, comportant en outre :
un moyen de commande de pression de gaz (37) pour commander de façon variable la pression du gaz provenant de la source (13) de gaz pressurisé en permanence par des signaux de commande audibles.

3. Appareil selon la revendication 2, comportant en outre un moyen d'indication (38), en liaison avec ledit moyen de commande de pression de gaz (37), pour délivrer une indication audible humainement de la pression du gaz provenant de la source de gaz pressurisé permanente (13).

4. Appareil selon la revendication 2, dans lequel ledit moyen de commande de pression de gaz (37) pour commander de façon variable la pression du gaz provenant de la source (13) de gaz pressurisé permanente délivre un signal audible confirmant un signal de commande audible détecté.

5. Appareil selon la revendication 4, comportant en outre un moyen de commande audible de position de soupape relié aux moyens de distribution (26) pour commander sélectivement la liaison soit du second (24), soit du troisième (27) conduit avec l'instrument d'infusion chirurgicale oculaire (29) par des signaux de commande audibles.

6. Appareil selon la revendication 5, comportant en outre un moyen de rétroaction pour accuser réception des moyens de commande audibles de position de soupape.
